# EUROPEAN PATENT APPLICATION

(11) **EP 2 656 876 A1**
(43) Date of publication of application: **30.10.2013**
(21) Application number: 12165492.5
(22) Date of filing: 25.04.2012
(51) Int. Cl.: A61N 1/37, A61N 1/08

(54) **A thin film for a lead for neural applications**

(71) Applicant: Sapiens Steering Brain Stimulation B.V., 5656 AE Eindhoven (NL)
(72) Inventor: Tol, Jeroen Jacob Arnold, 5645 JG Eindhoven (NL); Harberts, Dick Willem, 5627 MZ Eindhoven (NL); Young, Edward Willem Albert, 6211 LN Maastricht (NL); Bakker, Egbertus Johannes Maria, 4261 BX Wijk en Aalburg (NL)
(74) Representative: Rupprecht, Kay

(57) **Abstract**

The present invention relates to a thin film (301) for a lead (300), especially for a lead (300) for brain applications, preferably for a lead (300) for a neurostimulation and/or neurorecording system, whereby the thin film (301) comprises a proximal end (310) and a distal end (304), whereby the thin film (301) further comprises at least one extension means (320) at the distal end (304). Furthermore, the present invention relates to a lead, a probe and a neurostimulation and/or neurorecording system.

## Description

The present invention relates to a thin film for a lead, especially for a lead for neural applications, a lead, a probe and a neurostimulation and/or neurorecording system.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a stylet material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

Typically, the MR compatibility of probes is an issue that should be addressed. Serious field enhancement may occur as a result of the electrical field during scanning. The high field gives rise to tissue heating. It is common practice to coil the lead wires to create inductance. The inductance limits the creation of strong electric fields in the brain during MR scanning and subsequent heating in these areas of the brain. However, field gradient enhancement near the probe, in particular at the distal-end, is still an issue, also because the distal-end of the electrode is not coiled and therefore it is not an integral part of the inductor. Moreover, the field at the probe tip is dominant because the enhancement gradient is most pronounced in this area and most heating of tissue is expected at location in close vicinity of the distal-end in particular.

Heating of the tissue as a result of high electric field strength near the probe during MRI scanning is a serious risk. In particular, strong fields may be generated at the lead distal end.

It is therefore an object of the present invention, to improve a thin film for a lead, a probe, and a neurostimulation and/or neurorecording system, particularly in that the heating of the tissue as a result of high electric fields strength near the lead during MRI scanning is lowered.

The above object is solved according to the present invention by a thin film for a lead according to claim 1. Accordingly, a thin film for a lead is provided, whereby the thin film comprises a proximal end and a distal end and whereby the thin film further comprises at least one functional extension means at the distal end.

The thin film can be e.g. a thin film for a lead for neural applications or, more specifically for brain applications, preferably for a lead for a neurostimulation and/or neurorecording system. The neurostimulation and/or neurorecording system can be e.g. a DBS system. The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or merely the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which remote to the burr-hole of the skull, through which the lead is implanted.

According to the present invention a modification and reduction of the electric-field enhancement in the close vicinity of the distal end is achieved by the design of the thin film pattern in the distal end of the thin film probe. The present invention targets the field around the lead tip. To this end, a functional extension is added on the thin film probe to reduce the enhancement of the electrical field at the lead tip. Thereby, heating of the tissue as a result of high electric fields strength near the lead during MRI scanning is lowered due to the by fact that by adding an functional extension means to the distal end of the thin film due to the fact that the resulting density of equipotential lines can be lowered by the functional extension means. The functional extension means may in general be configured to influence the distribution of the electric field which emerges from the distal end of the thin film. In particular, the functional extension means may be adapted to reduce the enhancement of the electric field which may surround the distal end of the thin film.

Additionally, it is possible that the thin film comprises a plurality of electrodes arranged at the distal end of the thin film and that the lead comprises a distal lead tip and that the functional extension means is arranged and/or arrangeable between the plurality of electrodes and the distal lead tip.

Moreover, the functional extension means may comprise at least one thin film conductor. Thereby, a high resistant lead end can be realized.

It is possible that the functional extension means comprises at least partially a resistive material having an ohmic resistivity. The resistivity may be relatively large. For example, substochiometric Silicon Oxide or Silicon Nitride or Titanium Oxide may be applied. The preferred resistivity is in the order of 10 S/m. In particular, the resistivity is selected at a value which is at least larger than the resistivity of a material used for conducting current and thus having a good electrical conductivity and a low resistivity. By using a resistive material having an ohmic resistivity for the functional extension means, the field gradient induced during magnetic resonance imaging at the distal end of the thin film and thus at the distal end of the lead can be decreased. By providing a functional extension means adjacent to the distal tip end of the lead the equipotential lines of the field being existent during magnetic resonance imaging can cross the region of the resistive material having an ohmic resistivity, which is not the case when there is no region with relatively high resistivity. Since these equipotential lines do not have to bend around the tip of the lead, the resulting density of equipotential lines can lowered. This results in lower electric field strength at the tip end of the lead and thus the risk of increasing the temperature at the tip end is significantly lowered.

It is additionally possible that the functional extension means is configured such that it yields impedance when exposed to frequencies used during magnetic resonance imaging. Thereby, a similar effect can be achieved like using a functional extension means comprising at least partially a resistive material having an ohmic resistivity. Similarly, thereby the field gradient induced during magnetic resonance imaging at the distal end of the thin film and thus at the distal end of the lead can be decreased. Since the equipotential lines do not sharply bend around the tip of the lead but are spread out, the resulting density of equipotential lines can lowered. This results in lower electric field strength at the tip end of the lead and thus the risk of increasing the temperature at the tip end is significantly lowered.

Moreover, the functional extension means may comprise at least partially a resistive section having a capacitive resistivity and/or that the functional extension means comprises at least one capacitive coupling means having a capacitive resistivity.

An option to realise impedance on the functional extension connected to the thin film is by means of a capacitor ring. Thereby, a similar effect can be achieved like using a functional extension means comprising at least partially a resistive material when exposed to frequencies used during magnetic resonance imaging. Similarly, thereby the field gradient induced during magnetic resonance imaging at the distal end of the thin film and thus at the distal end of the lead can be decreased and the resulting density of equipotential lines can lowered. This results also in lower electric field strength at the tip end of the lead and thus the risk of increasing the temperature at the tip end is significantly lowered.

The functional extension means comprises a plurality of capacitors.

For instance, a (weak) capacitive coupled ring pattern can be applied. Preferably, the capacitance of coupled ring pattern is in the order of 20 pF. The resistor in the functional extension means may also be capacitively coupled to the electrodes of the lead at MR frequencies.

Appropriate coupling can be achieved by using a double metal structure in the distal part of the thin film. The extension capacitor or resistive part may be coupled to the distal electrodes by means of a capacitive coupling.

Generally, it is possible to combine several or all of the above possibilities to increase the resistivity and/or impedance of the functional extension means to decrease the field gradient induced during magnetic resonance imaging at the distal end of the thin film and thus at the distal end of the lead. In fact, the advantageous effect is not limited to ohmic resistors alone, but can be achieved by any structure that yields impedance at MR frequencies. Moreover, the high frequency impedance can also be realized as a capacitor.

It is an further possibility that the functional extension means is arranged and/or configured such that it comprises at least partially a resistivity and/or impedance gradient. By providing a gradient, the effectiveness of a resistive functional extension means to the distal end of the lead can be further enhanced.

For instance, it is possible that the resistivity and/or impedance gradient is configured such that the functional extension means comprises at least a first resistivity and/or impedance at the one end of the functional extension means and at least a second resistivity and/or impedance at a second end of the functional extension means, whereby the first resistivity and/or impedance is higher than the second resistivity and/or impedance.

Moreover, it is possible that the first end of the functional extension means is arranged near to the distal lead tip and the second end of the functional extension means is arranged next to the electrodes. This alignment may advantageously prevent the development of strongly enhanced gradients of the field at the side of the probe, near to the electrode area.

Additionally, the functional extension means comprises at least one thin film metal film. Thereby, a relatively high resistant functional extension can be provided. Dual metal configurations with partial overlap may also be applied. In particular, a configuration of at least two partially overlapping films of different metals can be applied for creating a capacitive coupling between the functional extension means and the distal end of the lead.

Furthermore, it is possible that the functional extension means is at least partially structured in a grid like pattern. By providing a functional extension means that is at least partially or e.g. merely in total structured in a grid like pattern, the resistivity of the functional extension means can be advantageously increased.

Moreover, the present invention relates to a lead with the features of claim 13. Accordingly, a lead is provided, which comprises at least one thin film according to any of claims 1 to 12. Especially, the lead may be a lead for neural applications, more specifically for brain applications, preferably a lead for a neurostimulation and/or neurorecording system.

Additionally, the present invention relates to a probe with the features of claim 14. Accordingly, a probe is provided, which comprises at least one thin film according to any of claims 1 to 12 and/or a lead according to claim 13. Especially, the probe may be a probe for neural applications, more specifically for brain applications, preferably a probe for a neurostimulation and/or neurorecording system. Such a probe may comprise a lead and an Advanced Lead Connector (ALC) element comprising electronic means to address electrodes on the distal end of the lead.

Furthermore, the present invention relates to a neurostimulation and/or neurorecording system with the features of claim 15. Accordingly, a neurostimulation and/or neurorecording system is provided, which is comprising at least one thin film according to any of claims 1 to 12 and/or a lead according to claim 13 and/or a probe according to claim 14. The neurostimulation and/or neurorecording system may be especially a deep brain stimulation (DBS) system.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe neurostimulation system for deep brain stimulation (DBS) and its components;
- Fig. 3:: a schematical drawing of a probe system according to the present invention;
- Fig. 4:: a schematical drawing of the field enhancement around a conducting lead in a brain in an electric field during an MRI scan;
- Fig. 5:: a diagram regarding the relation between the electric field enhancement at 64 MHz;
- Fig. 6:: a schematical drawing showing the field enhancement around a conducting body with varying resistivity in an electric field;
- Fig. 7a, b:: schematical drawings regarding the field distribution around a lead in an electric field during an MRI scan;
- Fig. 8a-c:: schematical drawings of a state of the art thin film distal end design;
- Fig. 9a-c:: a schematical drawing of a thin film distal end design according to the present invention;
- Fig. 10:: a schematical drawing of a first embodiment of a functional extension means according to the present invention;
- Fig. 11:: a schematical drawing of a first embodiment of a functional extension means according to the present invention;
- Fig. 12:: a schematical drawing of a first embodiment of a functional extension means according to the present invention; and
- Fig. 13:: a schematical drawing of a distal lead end including a thin film with a functional extension according to the present invention.

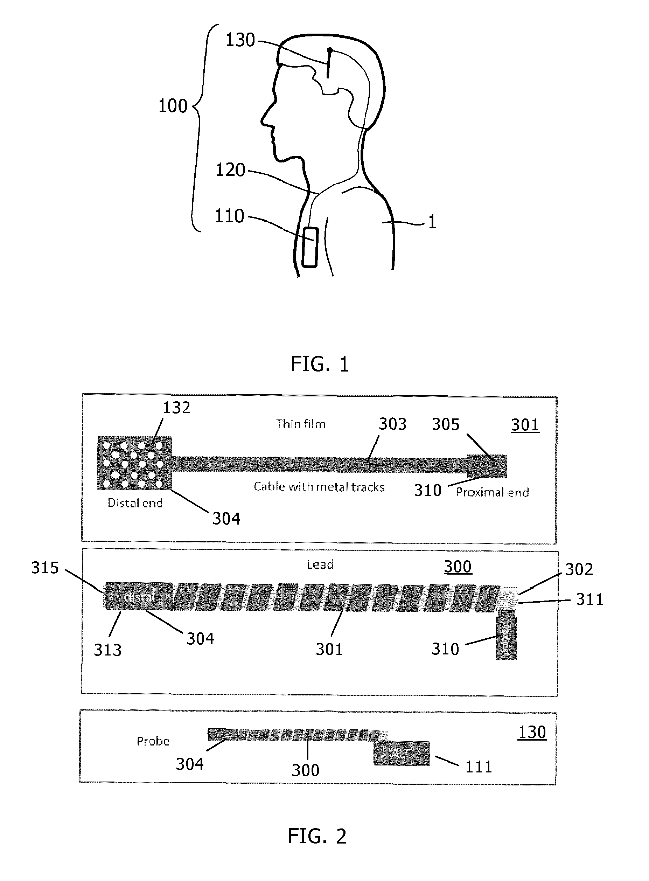

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Connector (ALC) element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the ALC element 111. The ALC element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

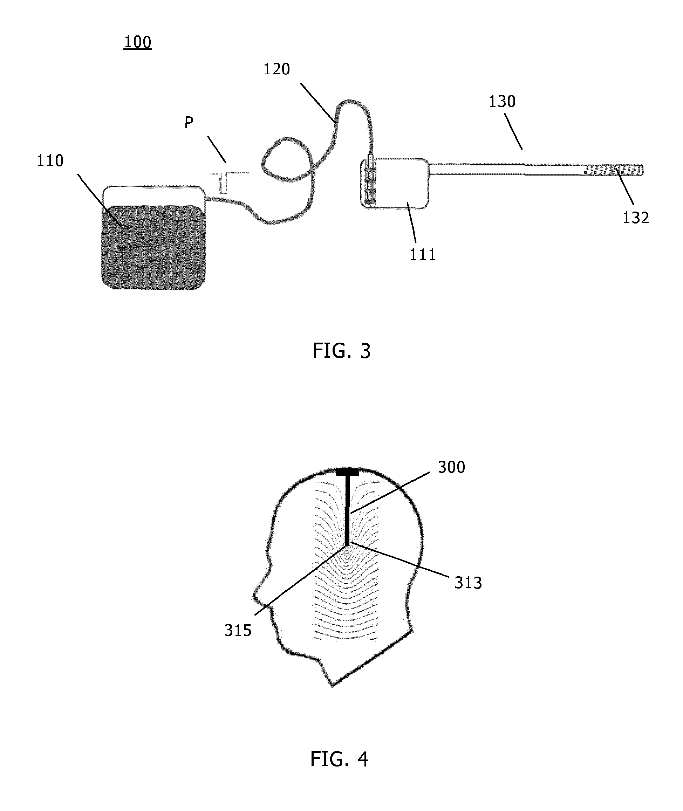

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 111. The controller 110 can be an implantable pulse generator (IPG) 110.

Figure 4 shows a schematical drawing of the field enhancement around a conducting lead 300 in a brain in an electric field during an MRI scan. As can be seen in Figure 4, the density of the field lines is increased around the distal tip 315 of the distal end 313 of the lead 300.

As mentioned above, a serious field enhancement may occur as a result of the electrical field during MRI scanning. The density of the field lines at the distal tip 315 of the lead gives rise to tissue heating. As shown in Figure 2, the thin film 301 is wound and is therefore a structure with an inductance. The inductance limits the creation of strong electric fields in the brain during MR scanning and subsequent heating in these areas of the brain. However, field gradient enhancement near the lead 300, for instance at the distal end 313 and the distal tip 315, is still an issue, also because the distal end 313 of the lead 300 is not coiled and therefore it is not an integral part of the inductor. Moreover, the field at the distal lead tip 315 is dominant because the enhancement gradient is most pronounced in this area and most heating of tissue is expected at location in close vicinity of the distal end 313.

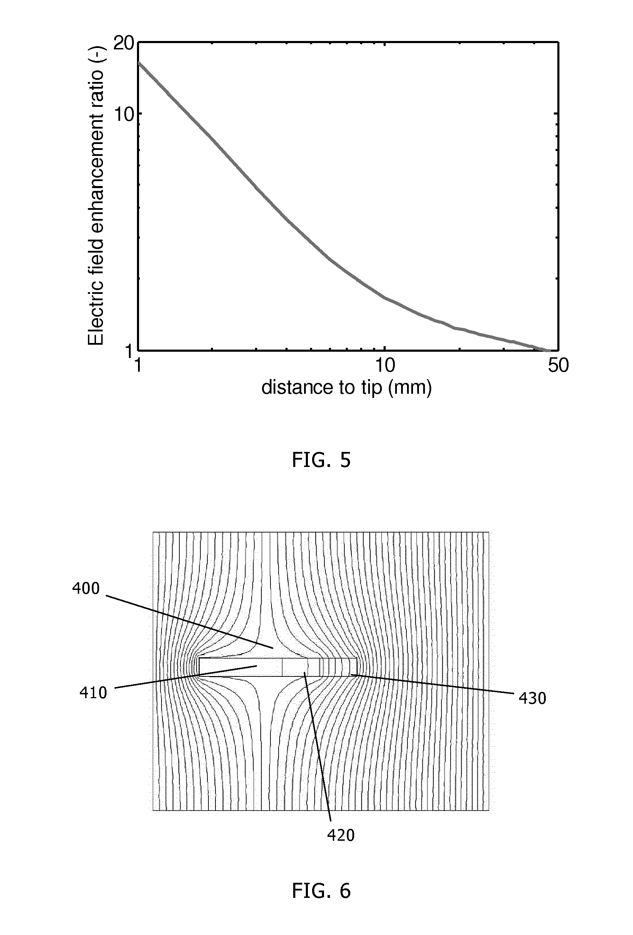

The correlation of this effect is shown in the diagram of Figure 5. This diagram is showing exemplarily a field gradient enhancement simulation at 64 MHz, particularly the correlation of the electric field enhancement ratio and the distance in mm to the distal tip 315 of the lead 300 with logarithmic scaling. As can be seen from the diagram, e.g. the electric field enhancement ratio is decreasing from a value of about 20 to a value below 10, when the distance to the tip is about 2 mm.

Figure 6 illustrates the effect of resistivity and shows vertical equipotential lines of the electric field component. The rectangle-formed bar 400 in the middle is divided into three regions 410, 420, 430 with different conductivities.

The left-hand side of Figure 6 shows the field distribution near a standard distal end of lead with electrodes which is formed by left-hand region 410 in this embodiment. The left-hand region 410 is highly conductive and has a low resistivity. The equipotential lines cannot enter such a material and thus the region 410 and are bent around its shape. Therefore, the density of the equipotential lines at the left-hand side of the bar 400 around region 410 is relatively high. This results in high electric field strength there because the electric field strength is proportional to the density of the equipotential lines or in other words the electric field equals the space derivative of the electric potential field.

The right-hand side of Figure 6 shows the effect of a high resistive extension which is formed by right-hand region 420 in this embodiment. The right-hand region 430 has a much higher resistance so that equipotential lines can cross this region 430. Because these equipotential lines do not have to bend around the right-hand side anymore, the resulting density of equipotential lines is much lower at the right-hand side of the bar 400 than at the left-hand side. This results in lower electric field strength at the right-hand side, near the region of high resistance, than at the left-hand side, near the region of low resistance.

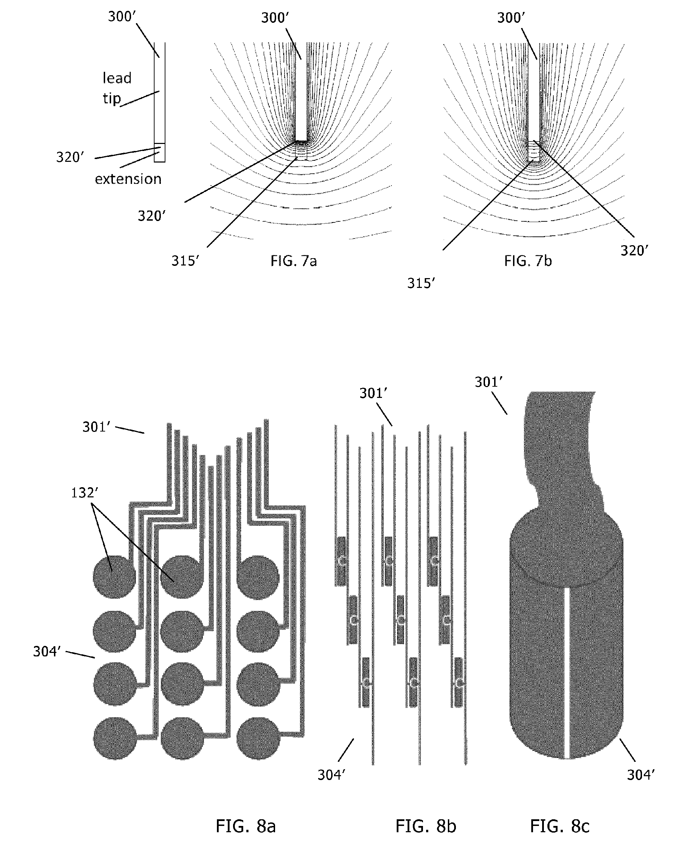

Figure 7a, b show the calculated effect of a functional extension on the electrical field in the vicinity of the tip 315', 315 of a lead 300', 300 in MRI scanning. The electric field strength is proportional to the density of the equipotential lines. As can be seen in Figure 7a, the field around the functional extension 320' next to the tip 315' of the lead 300' without conductivity is much more pronounced. As shown in Figure 7b, the field around the functional extension 320 next to the tip 315 of the lead 300 with appropriate conductivity is much smoother and will reduce the risk of heating of the brain tissue during MR scanning operations.

Figures 8a-c show schematical drawings of a state of the art thin film distal end design. As can be derived from Figure 8a, the distal end 304' of the thin film 301' comprises several electrodes 132' (see also Figure 2). Figure 8b shows the electrical model of the distal end 304' of the thin film 301'. Furthermore, Figure 8c shows a schematical perspective view of the distal end 304' of the thin film 301' and shows in particular, how the flat distal end 304' is folded around a round stylet (which is not shown). With the exception of the distal electrodes 132', all metal may be covered with dielectric material.

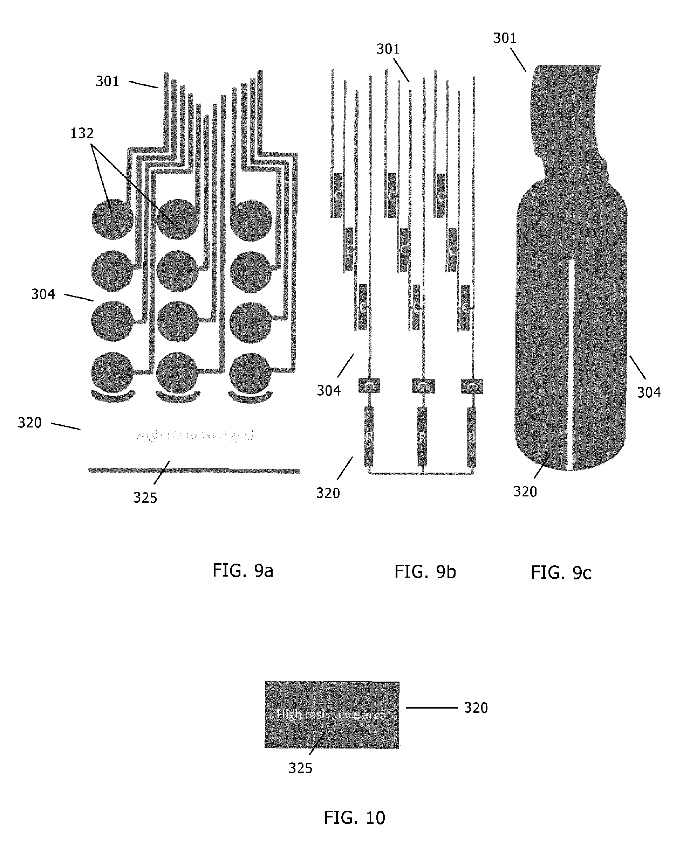

Figures 9a-c show schematical drawings of a thin film distal end design according to the present invention. As can be derived from Figure 9a, the distal end 304 of the thin film 301 comprises several electrodes 132 (see also Figure 2, the thin film 301 shown in Figure 9 comprises all features as the thin film 301 shown in figure 2 and as described above).

Particularly, the thin film 301 comprises a proximal end 310 (not shown in Figures 9a-c, see therefore Figure 2) and a distal end 304. The thin film 301 further comprises an functional extension means 320 at the distal end 304. The functional extension means 320 is arranged between the plurality of electrodes 132 and the distal lead tip 315 (see in particular Figure 9c and Figure 2).

Figure 9b shows the electrical model of the distal end 304 of the thin film 301. Furthermore, Figure 9c shows a schematical perspective view of the distal end 304 of the thin film 301 and shows in particular, how the flat distal end 304 is folded around a round stylet (which is not shown) together with the thin film functional extension means 320, which is in the shown embodiment formed by a thin film metal film. The so formed resistor in the functional extension means 320 is capacitively coupled to the electrodes 132 at MR frequencies.

By present invention the modification and reduction of the field gradient enhancement in the close vicinity of the distal end 304 of the thin film 301 pattern at the distal end 313 of the thin film lead 300 is achieved (see Figures 2 and 9a-c). A resistive element may be added to the thin film 301 at its the distal end 304 to decrease the field gradient. Consequently and advantageously, the heating of the tissue as a result of high electric field strength near the lead 300 during MRI scanning can be avoided, since strong fields are no longer generated at the distal end 313 of the lead 300.

By adding the functional extension means 320 to the thin film 301 and thus to the distal end 313 of the lead 300 the enhancement of the electrical field at the lead tip 315 may be significantly reduced. Moreover, the functional extension means 320 can be integrated on the thin film design, which is improving the manufacturing process and reducing the complexity of the manufacturing process.

A galvanic contact between distal electrodes 132 and the functional extension means 320 may be used but is not recommended. The functional extension means 320 may be covered with an insulator and will not affect the function of the DBS system. However, at the MRI frequencies, the functional extension means 320 may be capacitively coupled to the distal electrodes 132. The intended brain stimulation conducted with the DBS system 100 comprising the probe 130 with the lead 300 (see Figure 3) in normal operation occurs at low frequency and the functionality of the distal electrodes 132 is therefore not affected by the addition of the functional extension means 320. If needed, overlapping dual metal structures may be applied to create the appropriate capacitive coupling.

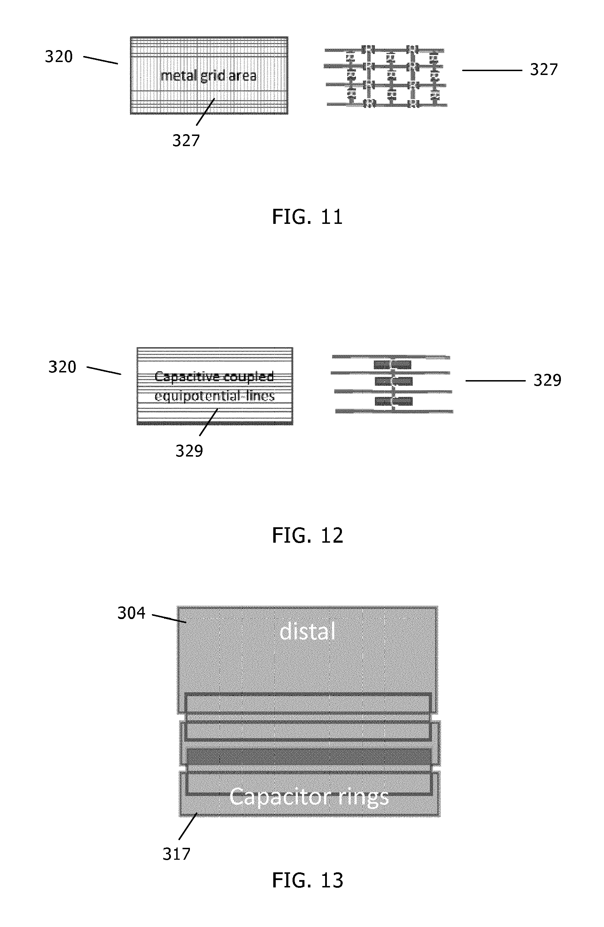

To create sufficient capacitive coupling between the capacitor rings 317, a dual metal layer thin film arrangement may be applied, as shown in Figure 13. Rings 317 in subsequent metal layers may overlap.

The gradient in the electric field is made smaller by means of an added, high resistive, mm's wide extension area of the distal end 304 of the thin film 301. This high resistant lead-end can be realized with a thin film conductor on the thin film lead 300 in an extension area of the distal end 313 of the lead, respectively the distal end 304 of the thin film 301.

This high resistant functional extension 320 can be realized by various means. A resistive material 325 can be applied in this area (see Figure 10) or a thin metal film (see Figure 9c) can be applied.

Moreover, to increase the resistivity, the film can be structured in a grid like pattern, e.g. by providing a metal grid area 327 (see Figure 11).

Alternatively to a resistor, a structure with high impedance at the MRI frequency can be applied. For instance, a weak capacitive (i.e. 20 pF) coupled ring pattern can be applied.

In fact, the effect is not limited to ohmic resistors, but any structure that yields impedance at MR frequencies. As shown in Figure 12, the high frequency impedance can therefore be realized as a capacitor 329.

The effectiveness of this resistive extension to the distal end 313 of the lead 300 can be further enhanced by applying a gradient in the resistivity of this extension 320. A relatively low resistivity at the electrode end and a higher resistivity towards the lead tip 315. This alignment will prevent the development of strongly enhanced gradients of the field at the side of the lead 300, near the electrode area.

## Claims

1. A thin film (301) for a lead (300), especially for a lead (300) for neural applications, preferably for a lead (300) for a neurostimulation and/or neurorecording system, whereby the thin film (301) comprises a proximal end (310) and a distal end (304) and whereby the thin film (301) further comprises at least one functional extension means (320) at the distal end (304).

2. The thin film (301) according to claim 1,
characterized inthat
the thin film (301) comprises a plurality of electrodes (132) arranged at the distal end (304) of the thin film (301) and that the lead (300) comprises a distal lead tip (315) and that the functional extension means (320) is arranged and/or arrangeable between the plurality of electrodes (132) and the distal lead tip (315).

3. The thin film (301) according to claim 1 or 2,
characterized inthat
the functional extension means (320) comprises at least one thin film conductor.

4. The thin film (301) according to any of the preceding claims,
**characterized in that**
the functional extension means (320) comprises at least partially a resistive material having an ohmic resistivity.

5. The thin film (301) according to any of the preceding claims,
**characterized in that**
the functional extension means (320) is configured such that it yields impedance (Z) when exposed to frequencies used during magnetic resonance imaging.

6. The thin film (301) according to any of the preceding claims,
**characterized in that**
the functional extension means (320) comprises at least partially a resistive section having a capacitive resistivity and/or that the functional extension means (320) comprises at least one capacitive coupling means having a capacitive resistivity.

7. The thin film (301) according to claim 6,
**characterized in that**
the functional extension means (320) comprises a plurality of capacitors.

8. The thin film (301) according to any of claims 4 to 7,
**characterized in that**
the functional extension means (320) is arranged and/or configured such that it comprises at least partially a resistivity and/or impedance gradient.

9. The thin film (301) according to claim 9,
**characterized in that**
the resistivity and/or impedance gradient is configured such that the functional extension means (320) comprises at least a first resistivity and/or impedance at the one end of the functional extension means (320) and at least a second resistivity and/or impedance at a second end of the functional extension means (320), whereby the first resistivity and/or impedance is higher than the second resistivity and/or impedance.

10. The thin film (301) according to claim 9,
**characterized in that**
the first end of the functional extension means (320) is arranged near to the distal lead tip (315) and the second end of the functional extension means (320) is arranged next to the electrodes (132).

11. The thin film (301) according to any of the preceding claims,
characterized inthat
the functional extension means (320) comprises at least one thin film metal film.

12. The thin film (301) according to any of the preceding claims,
characterized inthat
the functional extension means (320) is at least partially structured in a grid like pattern.

13. A lead (300), especially a lead (300) for neural applications, preferably a lead (300) for a neurostimulation and/or neurorecording system, comprising at least one thin film (301) according to any of claims 1 to 12.

14. A probe (130), especially a probe (130) for neural applications, preferably a probe (130) for a neurostimulation and/or neurorecording system, comprising at least one thin film (301) according to any of claims 1 to 12 and/or a lead (300) according to claim 13.

15. A neurostimulation and/or neurorecording system (100), especially a deep brain stimulation (DBS) system (100), comprising at least one thin film (301) according to any of claims 1 to 12 and/or a lead (300) according to claim 13 and/or a probe (130) according to claim 14.
